# EUROPEAN PATENT SPECIFICATION

(11) **EP 4 547 114 B1**
(45) Date of publication and mention of the grant of the patent: **14.10.2026**
(21) Application number: 23738143.9
(22) Date of filing: 13.06.2023
(51) Int. Cl.: A61B 8/12, A61B 8/08

(54) **SYSTEMS AND METHODS FOR CAVITY IMAGING IN PATIENT ORGAN BASED ON POSITION OF 4D ULTRASOUND CATHETER**
SYSTEME UND VERFAHREN ZUR HOHLRAUMBILDGEBUNG IN EINEM PATIENTENORGAN AUF BASIS DER POSITION EINES 4D-ULTRASCHALLKATHETERS
SYSTÈMES ET PROCÉDÉS D'IMAGERIE DE CAVITÉ DANS UN ORGANE DE PATIENT SUR LA BASE DE LA POSITION D'UN CATHÉTER ULTRASONORE 4D

(30) Priority: 29.06.2022 US 202217852952
(43) Date of publication of application: 07.05.2025
(73) Proprietor: Biosense Webster (Israel) Ltd., 2066717 Yokneam (IL)
(72) Inventor: GOVARI, Assaf, 2066717 Yokneam (IL); ALTMANN, Andres Claudio, 2066717 Yokneam (IL); GOLDBERG, Aviva, 2066717 Yokneam (IL)
(74) Representative: Carpmaels & Ransford LLP
(86) International application number: PCT/IB2023/056069
(87) International publication number: WO 2024/003646

(56) References cited:
- US-A1- 2020 107 886

## Description

### FIELD OF THE INVENTION

The present invention relates generally to medical imaging, and particularly to methods and systems for imaging using a four-dimensional (4D) ultrasound catheter.

### BACKGROUND OF THE INVENTION

Various techniques for imaging a cavity in an organ of a patient have been published.

For example, U.S. Patent Application Publication 2019/0053708 describes catheterization that is carried out by inserting a probe having a location sensor into a body cavity, and in response to multiple location measurements identifying respective mapped regions of the body cavity. Using the location measurements, a simulated 3-dimensional surface of the body cavity is constructed. One or more unmapped regions are delineated by rotating the simulated 3-dimensional surface about an axis. The simulated 3-dimensional surface of the body cavity is configured to indicate locations of the unmapped regions based on the location measurements.

U.S. Patent 10,163,252 describes systems and methods of automatically controlling on a graphical user interface used by a physician, display views of an anatomic structure of a patient. Such systems and methods of automatically controlling display views of an anatomic structure of a patient can facilitate visualizing a position of a medical device relative to the anatomic structure during a medical procedure directed to the anatomic structure. In certain implementations, the systems and methods of the present disclosure provide automatic display views of a cardiac catheter relative to a three-dimensional model of a patient's heart cavity during a medical procedure such as cardiac ablation.

US2020/107886A1 describes a system that includes a medical probe for insertion into a cavity of an organ, which includes a position and direction sensor and a camera, both operating in a sensor coordinate system. The system further includes a processor configured to: receive, from an imaging system operating in an image coordinate system, a three-dimensional image of the cavity including open space and tissue; receive, from the medical probe, signals indicating positions and respective directions of the medical probe inside the cavity; receive, from the camera, respective visualized locations inside the cavity; register the image coordinate system with the sensor coordinate system so as to identify one or more voxels in the image at the visualized locations, and when the identified voxels have density values in the received image that do not correspond to the open space, to update the density values of the identified voxels to correspond to the open space.

The present invention, which is defined by the independent claims. will be more fully understood from the following detailed description of the examples thereof, taken together with the drawings in which:

### BRIEF DESCRIPTION OF THE DRAWINGS

Fig. 1 is a schematic, pictorial illustration of a catheter-based ultrasound imaging system, in accordance with an example of the present invention;
Figs. 2A and 2B are schematic, pictorial illustrations of ultrasound images produced using system 20 of Fig. 1, in accordance with examples of the present invention; and
Fig. 3 is a flow chart that schematically illustrates a method for improving the quality of displayed ultrasound images, in accordance with an example of the present invention.

### DETAILED DESCRIPTION OF EXAMPLES

### OVERVIEW

Ultrasound imaging may be carried out in-vivo by inserting a four-dimensional (4D) ultrasound catheter into an organ in question, such as a chamber of a patient's heart. In principle, the 4D US catheter may provide real time ultrasound (US) images of a volume within a field-of-view (FOV) of the US image. For example, in response to applying ultrasound (US) waves to a heart cavity (e.g., atrium and/or ventricle),

The applied ultrasound waves penetrate through hollow cavities of the chamber and reflect from the chamber walls and surrounding organs within the field of view. Thus, when displaying an US image of the heart cavity, the tissue of the chamber wall is expected to appear in gray and the cavities are expected to appear in black. In other words, a gray color is assigned to the pixels (e.g., two-dimensional pixels or volumetric pixels referred to herein as voxels) of the US image at the positions that correspond to the chamber wall.

Ambiguity arises when the cavity is filled with blood. Blood includes cellular elements suspended in an extracellular matrix. The cellular elements may reflex the US waves so that the pixels in a portion of the cavity in which blood was present may appear in gray. These gray pixels may not be easily differentiated from the pixels that represent the chamber walls and therefore are considered noise.

Examples of the present invention that are described hereinbelow provide improved techniques for reducing noise from US images while visualizing an organ of a patient, such as from within a cavity of a patient heart.

In some examples, a system comprises one or more catheters for insertion into an organ of a patient, and a processor.

In some examples, the catheters may comprise at least one of: (i) a mapping catheter configured to sense electrocardiogram (ECG) signals in the patient heart, and (ii) an ablation catheter having one or more ablation electrodes configured to apply ablation signals to tissue of the heart. The system further comprises a catheter having a distal end comprising a (4D) ultrasound catheter having ultrasound transducers (UT), which are configured to apply US waves to an organ in question (e.g., heart cavity), and to produce, based on US waves returned (e.g., reflected) from the cavity in question, one or more US signals indicative of the shape and morphology of the cavity and surrounding tissue in question.

In some examples, the UT are arranged in a two-dimensional (2D) array. Note that when using 4D ultrasound imaging techniques, the processor is configured to produce three-dimensional (3D) ultrasound-based images, which are presented over time corresponding to the locations visited by the distal end of the catheter in the organ in question (e.g., right atrium of the heart).

In some examples, the distal end of each of the catheters described above and/or a distal end of the catheter shaft, typically comprises a position sensor, which is configured to produce one or more position signals indicative of one or more respective positions of the respective distal end inside the cavity in question.

In some examples, the processor is configured to receive and record the US signals and the position signals received from the distal end of at least one of the inserted catheters, and to produce an ultrasound (US) image of the cavity and tissue surrounding the cavity (e.g., tissue of the wall surrounding the cavity). Note that the 4D catheter is configured to travel or be positioned within the chamber, and therefore, is not intended to cut through the tissue surrounding the cavity (in other cases, the 4D US catheter may be used in conjunction with tools for cutting through tissue, such as in procedures that require the formation of a transeptal passage). Moreover, the geometry (i.e., physical dimension) of the distal end of each catheter is known and stored in the system.

In some examples, based on the recorded position signals received from each catheter and the known geometry of each catheter, the processor is configured to identify in the US image a given pixel (or a group of given pixels), which corresponds to a given position visited by one or more of the distal ends of the respective catheter, including the distal end assembly of the 4D ultrasound catheter (e.g., while applying the US signals). In such examples, the processor is configured to tag the given pixel (or the group of given pixels) as a pixel of the cavity.

In case one or more of the given pixels of the cavity appear gray by mistake (as described above), the processor checks whether the position signals indicate that the one or more respective distal ends have visited the given position corresponding to the given pixel(s). In case the one or more respective distal ends have visited the given position, the processor is configured to tag the one or more given pixels as pixels of the cavity. For example, in case the a given pixel is tagged, by mistake, as a tissue pixel and appears gray, the processor is configured to assign a black color to the given pixel. Moreover, the processor is configured to display, e.g., on a display of the system, a revised US image in which the tagging of one or more pixels is amended based on the disclosed techniques.

In the context of the present disclosure and in the claims, the terms "distal end" and "distal end assembly" and grammatical variations thereof are used interchangeably and refer to the distal tip of one or more respective catheters.

The disclosed techniques improve the quality of ultrasound images of organs, which are obtained using 4D ultrasound catheters and additional catheters used during a medical procedure. Moreover, the disclosed techniques may be used, *mutatis mutandis,* in imaging using other sorts of imaging sensors, which are coupled together with one or more position sensors, to one or more distal end of one or more respective catheters.

### SYSTEM DESCRIPTION

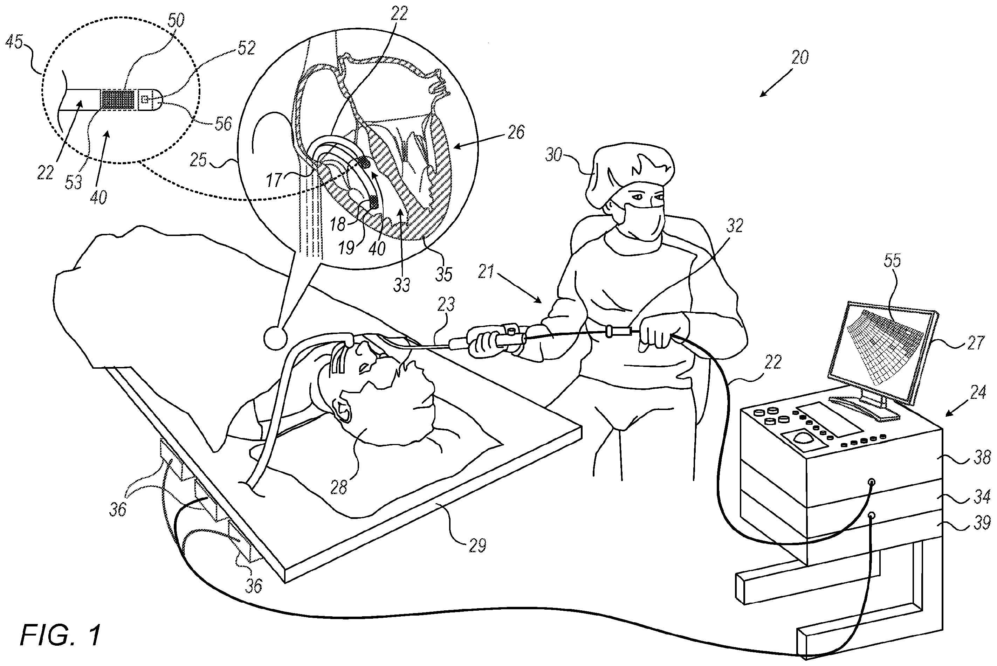

Fig. 1 is a schematic, pictorial illustration of a catheter-based ultrasound imaging system 20, in accordance with an example of the present invention.

Reference is now made to an inset 45. In some examples, system 20 comprises one or more catheters, such as but not limited to a catheter 21 having a distal end assembly 40 that comprises ultrasound transducers (UT) 53 that in the present example are arranged in a two-dimensional (2D) ultrasound array, also referred to herein as a 2D array 50 of the UT. Distal end assembly 40 further comprises a position sensor 52 coupled at a known position relative to 2D array 50.

In some examples, 2D array 50 is configured to apply ultrasound (US) waves to an organ, in the present example, a heart 26 of a patient 28, and to produce one or more US signals indicative of a surface topography and morphology of the respective tissue of heart 26.

In some examples, position sensor 52 is integrated with and is pre-calibrated with 2D array 50 of catheter 21.

In some examples, position sensor 52 is configured to produce one or more position signals indicative of one or more respective positions of distal end assembly 40 inside heart 26 of patient 28 lying on a surgical table 29, as will be described in more detail herein.

Reference is now made back to the general view of Fig. 1. In some examples, system 20 comprises a processor 39, which is configured, based on the position signals received from position sensor 52, to estimate the direction and the orientation of distal end assembly 40, and more specifically of 2D array 50 of the UT inside (a cavity of) heart 26.

In some examples, based on the position signals received from position sensor 52, processor 39 is configured to produce an US image of heart tissue by registering between ultrasound images that were acquired by 2D array 50, in respective sections of the tissue of heart 26.

In some examples, distal end assembly 40 is fitted at the distal end of a shaft 22 of catheter 21, which is is inserted through a sheath 23 into heart 26. The proximal end of catheter 21 is connected to a control console 24. In the example described herein, catheter 21 is used for ultrasound-based diagnostic procedures. In other examples, the catheter may be also used in therapeutic procedures, such as in electrical sensing and ablation of tissue in heart 26, using a tip electrode 56 shown in inset 45.

Reference is now made to an inset 25. In some examples, system 20 comprises an additional catheter 17 having a position sensor 18 and an electrode 19, both are coupled to the distal end of catheter 17 at known respective positions.

In some examples, position sensor 18 is configured to produce one or more position signals indicative of one or more respective positions of the distal end of catheter 17 inside heart 26.

In some examples, electrode 19 comprises a sensing electrode configured to sense electrocardiogram (ECG) signals at one or more positions in which the distal end of catheter 17 visits in heart 27. In other embodiments, electrode 19 comprises an ablation electrode configured to apply ablation signals to tissue at predefined location in heart 26.

In some examples, a physician 30 navigates distal end assembly 40 of catheter 21, and separately, the distal end of catheter 17 to respective target locations in a cavity 33 of heart 26 by manipulating shaft 22 using a manipulator 32 located near the proximal end of catheter 21. Note that cavity 33 is surrounded by tissue 35 and by ostia of vasculature connected to cavity 33. In the example of Fig. 1, physician 30 navigates distal end assembly 40 and the distal end of catheter 17 into cavity 33, e.g., a right atrium of heart 26, and applies the UT of 2D array 50 for producing US images of the right atrium.

Reference is now made back to insets 25 and 45. In some examples, physician 30 navigates 2D array 50 in cavity 33. In the present example, 2D array 50 comprises about 2048 UT 53 arranged in an array of about 64 columns and about 32 rows, and is configured to produce one or more US images of one or more respective sections of cavity 33 and tissue 35. Note that based on the position signals received from position sensor 52, the spatial coordinates of every pixel in the imaged section are known and calibrated for producing a full US image of the section in question. Note that the number of UT and the arrangement thereof in 2D array 50 is presented by way of example, and in other examples, 2D array 50 may comprise any other suitable number of UT 53 arranged in any similar or different suitable configuration.

In the context of the present disclosure and in the claims, the terms "about" or "approximately" for any numerical values or ranges indicate a suitable dimensional tolerance that allows the part or collection of components to function for its intended purpose as described herein.

Reference is now made back to the general view of Fig. 1. In some examples, control console 24 comprises processor 39, typically a general-purpose computer, with suitable front end and interface circuits 38 for receiving signals from catheter 21, as well as for, optionally, applying treatment via catheter 21 to tissue in heart 26 and for controlling the other components of system 20. Console 24 also comprises a driver circuit 34, configured to drive magnetic field generators 36.

In some examples, during the navigation of distal end assembly 40 in heart 26, console 24 receives position signals from position sensor 52 in response to magnetic fields from external field generators 36. Similarly, during the navigation of catheter 17 in heart 26, console 24 receives position signals from position sensor 18 in response to the magnetic fields applied by external field generators 36. In some examples, magnetic field generators 36 are placed at known positions external to patient 28, e.g., below table 29 upon which the patient is lying. The position signals are indicative of the position and direction of 2D array 50 in the coordinate system of a position tracking system, which is calibrated with the coordinate system of system 20. In the context of the present disclosure and n the claims, the terms "calibrated" and "registered" and grammatical variations thereof are used interchangeably.

The method of position sensing using external magnetic fields is implemented in various medical applications, for example, in the CARTO^{™} system, produced by Biosense Webster, and is described in detail in U.S. Patents 6,618,612 and 6,332,089, in PCT Patent Publication WO 96/05768, and in U.S. Patent Application Publications 2002/0065455, 2003/0120150, and 2004/0068178.

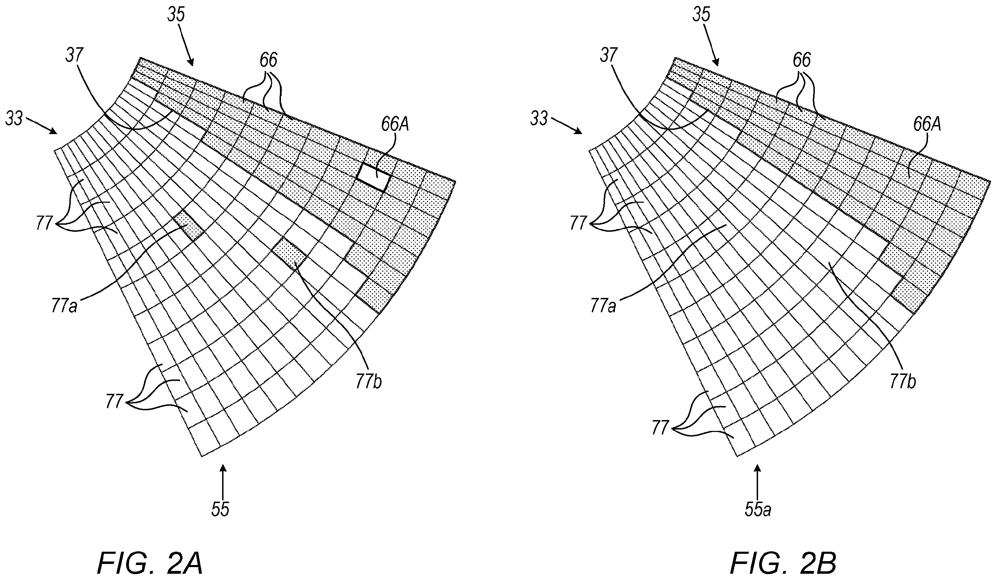

In some examples, processor 39 is configured to operate 2D array 50 by applying ultrasound waves to a respective section of heart 26 comprising at least cavity 33 and tissue 35, and sensing US waves returning from the respective section for imaging cavity 33 (e.g., the right atrium) and/or surrounding tissue (e.g., tissue 35) of heart 26. In an example, processor 39 is configured to display at least a section of the imaged cavity 33 to physician 30 on a display 27, e.g., as an ultrasound image, referred to herein as an image 55, or using any other suitable presentation. Examples related to image 55 are described in more details in Figs. 2A and 2B below.

In some examples, processor 39 typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

The example configuration shown in Fig. 1 is chosen by way of example for the sake of conceptual clarity. The disclosed techniques may be applied, *mutatis mutandis,* using other components and settings of system 20. For example, system 20 may comprise additional components and are configured to perform catheterization procedures other than cardiac.

### VISUALIZATION OF TISSUE AND CAVITY USING 4D ULTRASOUND CATHETER

Fig. 2A is a schematic, pictorial illustration of ultrasound image 55 produced using system 20 of Fig. 1, in accordance with an example of the present invention.

In some examples, physician 30 applies a four-dimensional (4D) ultrasound catheter, in the present example, distal end assembly 40, which is configured to produce 4D ultrasound data on tissue 35 and cavity 33. In the context of the present disclosure and in the claims, the term "4D ultrasound" refers to one or more ultrasound transducers, typically arranged in an array, which are configured to apply ultrasound (US) waves to an organ, and to produce one or more US signals indicative of three-dimensional (3D) features of the respective organ, and processor 39 is configured to produce US images based on the US signals. Note that each US image is a 2D image (of a slice of the organ in question) and processor 39 is configured to produce a 3D US image by integrating the 2D slices to a volumetric image having volumetric pixels (voxels). The fourth dimension is time. When physician 30 moves 2D array 50, processor 39 is configured to produce a video clip comprising the aforementioned 3D US images displayed over time, based on the respective positions of 2D array 50 that is moved within the organ in question (e.g., cavity 33 and tissue 35 of heart 26).

In the present example, physician 30 intends to perform an anatomical mapping of cavity 33 and tissue 35, e.g., for treating arrhythmia in heart 26 by applying radiofrequency (RF) ablation signals (e.g., pulses) to a section (not shown) of tissue intended to be ablated, or for any other suitable medical application. It will be appreciated that RF ablation is merely one of myriad therapeutic treatments in which US imaging may be useful. Pulsed-field ablation, sometimes referred to as irreversible electroporation (IRE) is another exemplary therapeutic treatment in which US imaging is useful.

In some examples, during the RF ablation procedure, a catheter, such as catheter 17, having one or more ablation electrodes (e.g., electrode 19) is inserted into the organ in question, and the ablation electrodes are placed in contact with the tissue intended to be ablated.

After obtaining sufficient contact force between each ablation electrode and the respective tissue, a user of an ablation system (e.g., physician 30) applies the RF ablation signals to the tissue. Note that during the RF ablation procedure, it is important to produce a continuous lesion along the entire section of the tissue intended to be ablated. In some cases, a topography in the surface of the section may cause insufficient contact between one or more of the ablation electrodes and the tissue intended to be ablated. Therefore, it is important to select a suitable position of the ablation electrodes when performing the RF ablation procedure.

In the present example, the organ in question is cavity 33 and tissue 35 of heart 26. In some examples, before performing the ablation procedure, physician 30 inserts: (i) the distal end of catheter 17 for performing the anatomical mapping, and (ii) distal end assembly 40 into cavity 33 and uses 2D array 50 for applying the US waves and producing one or more US signals indicative of the morphology (e.g., shape and surface topography) of at least a selected section of cavity 33 and tissue 35.

In some examples, the US signals are produced by using 2D array 50 for applying a 3D wedge (not shown) mode of acquisition that enables simultaneous acquisition of ultrasound images of the selected section of cavity 33 and tissue 35. As described in Fig. 1 above, 2D array 50 may comprise about 2048 UT 53 arranged in an array of about 64 columns and about 32 rows or any other suitable configuration.

In some examples, when physician 30 moves catheter 17 within cavity 33 (e.g., during the anatomical mapping and/or ablation of tissue 35), position sensor 18 generates position signals at respective visited 3D positions, and processor 39 is configured to record and store these positions, e.g., in a memory device and/or in processor 39 of system 20.

In some examples, based on the position signals from position sensor 52, processor 39 is configured to record 3D positions of the 4D US catheter (e.g., distal end assembly 40) while physician 30 moves the 4D US catheter within cavity 33, and acquires US signals using 2D array 50.

In some examples, based on the position signals from position sensors 18 and 52, the known dimensions of the distal-end assemblies of catheters 21 and 17, and the US signals, processor 39 is configured to calibrate between the coordinate systems of position sensor 52 and 2D array 50, and to identify the visited 3D positions as positions within cavity 33. Note that because catheters 17 and 21 cannot penetrate through tissue 33, every 3D position in which the distal-end assemblies of catheters 17 and 21 have visited must be within cavity 33. Subsequently, processor 39 is configured to produce US image 55. Based on the 3D position, the known dimensions of the respective distal end, and the ultrasound signals, processor 39 is configured to identify the voxels in US images 55 of heart 26, which correspond to the additional 3D position, and to associate these voxels with tissue 33. In other words, based on the known visited positions and geometrical dimensions, the distal ends and shafts of the catheters may be used as an eraser tool that erases all the gray areas that are not part of the chamber wall. Processor 39 is further configured to display one or more US images 55, such that distal end assembly 40, and when applicable, the distal end of catheter 17 are positioned within the field-of-view of US image 55. Thus, physician 30 can see the US image at the position that is currently visited by distal end assembly 40 and the distal end of catheter 17.

In some examples, based on the US signals of 2D array 50, the known dimensions of the distal ends of catheters 17 and 21, and the corresponding position signals of position sensors 18 and 52, processor 39 is configured to visualize the shape of cavity 33 and the surface of the selected section of tissue 35. The method of applying US signals and a position sensor for producing, *inter-alia,* ultrasound-based anatomical images is described in additional patent applications of the applicant, for example, in U.S. Patent Applications 17/357,231 and 17/357,303.

In some examples, processor 39 is configured to produce image 55 indicative of the shape of at least a section of cavity 33 and the topography and shape of the respective section of tissue 33. As described in Fig. 1 above, processor 39 is configured to display to physician 30 on display 27, image 55 having a wedge shape.

In some examples, image 55 comprises one or more pixels, in the present example volumetric pixels (voxels) 77, indicative of the imaging of cavity 33. In the present example, a first color is assigned to pixels 77. For example, processor 39 is configured to present pixels 77 in black color, which is obtained based on the interaction between the US waves and the blood that fills cavity 33 at a maximal expansion position thereof (e.g., in maximal expansion of the right atrium). Note that when 2D array 50 applies US waves, almost no US waves return from cavity 33 to US sensors of 2D array 50. Thus, processor 39 typically assigns a black color to pixels 77. In the example of Fig. 2A, because black pixels may not appear as a grid of pixels, pixels 77 are displayed in white color purely for the sake of clarity in presenting the examples of the present invention.

In some examples, image 55 comprises one or more pixels 66 indicative of the imaging of tissue 35. In the present example, some of the applied US waves are returned from tissue 35 to 2D array 50 (e.g., more than from cavity 33), thus, a second different color (e.g., gray) is assigned to pixels 66. Image 55 further comprises a line 37 (shown in boldface for the sake of conceptual clarity) representing the shape of the surface of tissue 35, which also represents the interface between cavity 33 and tissue 35. Any suitable color (e.g., white, light gray or black) may be assigned to line 37. For example, in case line 37 returns (e.g., reflects) to back 2D array 50 more US waves than tissue 35, then line 37 may appear in bright gray or in white color.

In some examples, pixels 66 and 77 (and any other pixels of image 55) may comprise 2D pixels or volumetric pixels (voxels) used for imaging the volume of tissue 35 and cavity 33, respectively.

In some examples, processor 39 is configured to gate the visualization of the section of cavity 33 and tissue 35 to a suitable phase of the cardiac cycle of beating heart 26, e.g., to the cardiac phase in which heart 26 is fully expanded or fully contracted. The gating is essential for providing physician 30 with the most accurate shape and surface topography of cavity 33 and tissue 35, without producing imaging artifacts related to different stages of the cardiac contraction of heart 26.

In some examples, processor 39 is configured to produce image 55 when physician 30 moves distal end assembly 40 (and the distal end of catheter 17) within cavity 33. In some cases, the color assigned to one or more of pixels 66 and 77 may be mistaken, for example, due to the presence of solid particles (e.g., fat or clot) within the blood or another substance, or due to the contraction phase of heart 26. In the example of image 55, gray color is assigned by mistake to pixels 77a and 77b of cavity 33, and white color is assigned by mistake to a pixel 66a of tissue 35.

### IMPROVING THE QUALITY OF ULTRASOUND IMAGE BASED ON POSITION SIGNALS RECEIVED FROM POSITION SENSOR

Fig. 2B is a schematic, pictorial illustration of an ultrasound image 55a, which is produced using system 20 and has improved quality compared to image 55 of Fig. 2A above, in accordance with an example of the present invention.

In some examples, based on the position signals from position sensors 19 and 52, processor 39 is configured to record 3D positions of the distal end of catheter 17 and of the 4D US catheter (e.g., distal end assembly 40) while physician 30 moves catheter 17 and the 4D US catheter within cavity 33, and acquires US signals using 2D array 50.

Based on the techniques described in Fig. 2A above, based on the position signals received from position sensors 19 and 52, the known physical dimensions of the distal end of catheters 17 and 21, and the US signals acquired by 2D array 50, processor 39 is configured to calibrate between the coordinate systems of position sensors 19 and 52 and 2D array 50. Subsequently, processor 39 is configured to produce images 55 and 55a of Figs. 2A and 2B, respectively. Based on the calibration, processor 39 is configured to display US image 55a, such that distal end assembly 40 (and when applicable also the distal end of catheter 17, are positioned within the field-of-view of US image 55a.

In some examples, based on the fact that distal end assembly 40 and the distal end of catheter 17 cannot perforate (e.g., cut through) tissue 35 (or any other tissue) of heart 26, processor 39 is configured to assign the white color to every voxel corresponding to a position signal received from position sensors 19 and 52. In other words, every position within heart 26, which is visited by distal end assembly 40 and the distal end of catheter 17, are considered a cavity (because distal end assembly 40 cannot pass through tissue 35). Thus, processor 39 is configured to classify all the voxels corresponding to the visited positions, as cavity voxels, and to assign a white color to these respective voxels. The color assignment is used for tagging each voxel so that physician 30 can immediately see on US image 55a (as well as on US image 55 of Fig. 2A above) whether the position of each voxel corresponds to tissue 35 or to cavity 33. In the context of the present disclosure and in the claims, the terms "tagging" and "display the voxel(s) as" and grammatical variations thereof are used interchangeably and refer to the presentation of one or more voxels on the respective US map, e.g., on display 27.

Similarly, in some case physician 30 cannot position distal end assembly 40 at a position having one or more voxels classified as cavity voxels (i.e., indicative of cavity 33). In some examples, processor 39 is configured to reclassify at least one of these one or more voxels as a tissue voxel and to tag the respective one or more voxels by assigning to them a gray color.

In the context of the present disclosure, the terms "cavity pixel" and "cavity voxel" refer to a pixel and a voxel, respectively, whose position corresponds to a position of a cavity (e.g., cavity 33) of heart 26. Similarly, the terms "tissue pixel" and "tissue voxel" refer to a pixel and a voxel, respectively, whose position corresponds to a position of tissue (e.g., tissue 35) of heart 26.

In the example of Fig. 2B, physician 30 moves distal end assembly 40 and/or the distal end of catheter 17, *inter alia,* at positions corresponding to pixels 77a and 77b. In some examples, in response to (i) identifying that pixels 77a and 77b correspond to the aforementioned visited positions, and (ii) identifying that a gray color assigned to pixels 77a and 77b (as shown in Fig. 2A above), processor 39 is configured to reclassify pixels 77a and 77b as cavity pixels and to assign a white color (instead to the gray color shown in image 55 above) to both pixels 77a and 77b.

In some examples, in response to (i) identifying that pixel 66a corresponds to a position that distal end assembly 40 and/or the distal end of catheter 17 cannot visit, and (ii) identifying that a white color assigned to pixel 66a (as shown in Fig. 2A above), processor 39 is configured to reclassify pixel 66a as a tissue pixel and to assign gray color to pixel 66a.

In such examples, based on one or more positions received from position sensors 19 and 52, processor 39 is configured to reclassify one or more pixels or voxels on image 55 and to produce image 55a having a different color assigned to the reclassified pixels. In other words, in response to receiving a position signal visited by distal end assembly 40 and/or the distal end of catheter 17, and identifying that a given pixel in the image corresponds to the visited position, processor 39 is configured to display the given pixel as a cavity pixel on display 27. Similarly, processor 39 may identify an additional position that cannot be visited (by distal end assembly 40 and/or by the distal end of catheter 17) as tissue, and may classify one or more pixels corresponding to the additional position as a tissue pixel and display the classified pixel(s) as tissue pixels having a gray color assigned thereto.

Images 55 and 55a of Figs. 2A and 2B, respectively, are shown by way of example, and are simplified for the sake of conceptual clarity. For example, images 55 and 55a comprise about 200 pixels each, whereas a typical ultrasound image may comprise millions of pixels. Thus, at least one of, and typically each of pixels 66, 66a, 77, 77a and 77b, comprises any suitable number of pixels, e.g., about 10,000 pixels. Moreover, processor 39 is configured to reclassify and alter the color to a single pixel or to a group of pixels located at any position of Figs. 2A and/or 2B. Moreover, processor 39 is configured to display on display 27 pixels or voxels in images 55 and 55a.

In other examples, each pixel or voxel of images 55 and/or 55a may comprise more than a cavity pixel or a tissue pixel. For example, in case an organ comprises several types of tissue having features different from one another, each tissue type may have a different classification. In such examples, processor 39 is configured to classify each pixel in accordance with the US signals received from 2D array and based on the position of each pixel or voxel using the examples described above in Figs. 2A and 2B. Moreover, processor 39 is configured to assign a different color to each pixel that corresponds to a different tissue type.

In alternative examples, instead of or in addition to assigning a color code in accordance with the classification of each pixel, processor 39 is configured to display the pixels or voxels using any other technique that visually differentiates between the different classes of the pixels. For example, different textures and/or different icons assigned to one or more pixels having common features or different features, such as tissue pixels and cavity pixels.

In some examples, when physician 30 moves distal end assembly 40 within cavity 33 between a first position and a second position, which is different from the first position, processor 39 is configured to receive: (i) first and second position signals indicative of the first and second positions, respectively, and (ii) first and second US signals indicative of cavity 33 and/or tissue 35 at the first and second positions, respectively. In some examples, based on the first and second position signals and the corresponding US signals, processor 39 is configured to present on display 27, at least first and second US images of the first and second positions, respectively. In some examples, the pixels comprise voxels, and processor 39 is configured to present 3D ultrasound images. Moreover, processor 39 is configured to present, e.g., on display 27, a video clip comprising at least the first and second 3D US images, and typically the video clip comprises US imaging of at least cavity 33 and/or tissue 35 that are located between the first and second positions.

### IMPROVING QUALITY OF 4D ULTRASOUND IMAGES DISPLAYED TO A USER

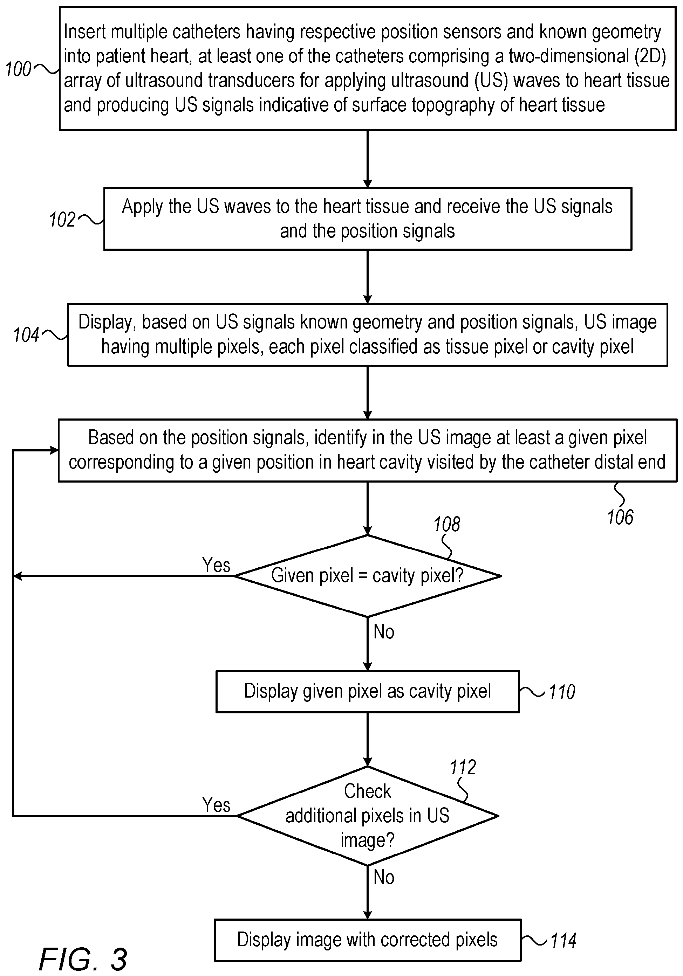

Fig. 3 is a flow chart that schematically illustrates a method for improving the quality of ultrasound image 55 displayed to physician 30, in accordance with an example of the present invention.

The method begins at a catheter insertion step 100, with inserting multiple catheters into the right atrium of heart 26, in the present example, inserting distal end assembly 40 and the distal end of catheter 17 into the right atrium of heart 26. Note that the geometry (i.e., physical dimensions) of the distal ends of both catheters are known and stored in system 20, e.g., in processor 39. As described, for example, in Fig. 1 above, the distal end of catheter 17 comprises magnetic position sensor 18 and electrode(s) 19, and distal end assembly 40 comprises 2D array 50 of UT and position sensor 52. In some examples, 2D array 50 is configured to: (i) apply US waves to tissue in question (e.g., tissue 35) and (ii) produce US signals indicative of the surface topography of the heart tissue in question. Position sensors 18 and 52 are configured to produce position signals indicative of the respective positions of the distal end of catheter 17 and distal end assembly 40 inside the right atrium (i.e., cavity 33) of heart 26.

In some examples, processor 39 is configured to calibrate between the coordinate systems of 2D array 50 and position sensor 52. Note that the calibration is typically carried out before the catheter insertion (e.g., in the production and/or qualification process of catheters 17 and 21) for reducing the time of the diagnostic procedure, but in other examples, the calibration or verification thereof may be carried out after the insertion of the distal end of catheter 17 and/or of distal end assembly 40 into cavity 33.

At an ultrasound application step 102, while physician moves distal end assembly 40 within cavity 33, processor 39 controls 2D array 50 to apply the US waves to tissue 35 and/or to cavity 33. In some examples, processor 39 receives the US signals (from 2D array 50) and the position signals (from position sensor 52), as described in detail in Figs. 1, 2A and 2B above.

At a first visualization step 104, based on the received US signals, the known geometry of the distal end of catheters 17 and 21, and the position signals received from position sensors 18 and 52, processor 39 produces US image 55 that has: (i) tissue pixels, such as pixels 66, and (ii) cavity pixels, such as pixels 77, as described in detail in Fig. 2A above. Note that each pixel may comprise a 2D pixel or a volumetric pixel (voxel) and may be classified and tagged in US image 55 as a tissue voxel (or pixel), or a cavity voxel (or pixel). Moreover, in the context of the present disclosure and in the claims, the term "pixel" may refer to a 2D pixel or a volumetric (3D) pixel, i.e., voxel.

At a pixel verification step 106, based on the position signals received from position sensors 18 and 52, processor 39 is configured to identify in US image 55 at least a given pixel corresponding to a given position in cavity 33, which was visited by the distal end of catheter 17 and/or by distal end assembly 40 while sensing ECG signals and/or while acquiring the US signals.

At a first decision step 108, processor 39 is configured to check whether the given pixel is classified and tagged as a cavity pixel.

In case the given pixel (e.g., pixel 77) of US image 55 is tagged as a cavity pixel, the method loops back to step 106 and processor 39 checks another pixel of US image 55, and at step 108 processor 39 checks whether the other pixel is classified and tagged as a cavity pixel.

In case the given pixel (e.g., pixel 77a) of US image 55 is tagged as a tissue pixel, the method proceeds to a tagging step 110. In some examples, in step 110 processor 39 verifies that the position signal received from one or both position sensors 18 and 52 correspond to the position of pixel 77a, which means that the distal end of catheter 17 and/or distal end assembly 40 has visited the position corresponding to that of pixel 77a. In such examples, at step 110, in response to the verification, processor 39 is configured to reclassify pixel 77a as a cavity pixel and to alter the tagging of pixel 77a from a tissue pixel to a cavity pixel, as shown and described in detail in Fig. 2B above.

In other examples, processor 39 is configured to apply step 106-110, *mutatis mutandis,* to positions that cannot be visited by distal end assembly 40. For example, processor 39 is configured to check whether the distal end of catheter 17 and/or distal end assembly 40 have visited the position corresponding to pixel 66a. In case the distal end of catheter 17 and/or distal end assembly 40 have not visited and/or cannot visit the position corresponding to pixel 66a, processor 39 is configured to reclassify pixel 66a as a tissue pixel and to alter the tagging of pixel 66a from a cavity pixel to a tissue pixel, as shown and described in detail in Fig. 2B above. Note that the same technique is applied to pixel 77b, as described in detail in Fig. 2B above.

At a second decision step 112, processor 39 checks whether additional pixels of the US image (e.g., image 55) must be checked. In case there are additional pixels that must be checked, the method loops back to step 106.

In case no additional pixels of US image 55 must be checked, the method proceeds to a second visualization step 114 that concludes the method. In step 114, processor 39 is configured to display to physician 30, e.g., on display 27, image 55a in which the tagging of pixels 66a, 77a and 77b is corrected using the techniques described in Fig. 2B and in steps 106-110 above.

The method of Fig. 3 is intended to improve the quality of US images displayed to physician 30 and may be carried out during the mapping of heart 26 (or any other organ) as well as during a therapeutic medical procedure, such as during the tissue ablation procedure. Note that as long as the position signals are recorded, processor 39 may reclassify and retag pixels of the US image(s) at any suitable time, e.g., even offline.

Moreover, the method of Fig. 3 is simplified for the sake of conceptual clarity, and typically comprises additional steps that are essential to carry out the visualization of the heart or that of any other suitable organ in question.

Although the examples described herein mainly address electro-anatomical mapping, tissue ablation and 4D US imaging of a patient heart, the methods and systems described herein can also be used in other patient organs and/or in other applications.

It will thus be appreciated that the examples described above are cited by way of example, and that the present invention is not limited to what has been particularly shown and described hereinabove. Rather, the scope of the present invention is defined by the independent claims and may include both combinations and sub-combinations of the various features described hereinabove, as well as variations and modifications thereof which would occur to persons skilled in the art upon reading the foregoing description and which are not disclosed in the prior art.

## Claims

1. A system, comprising:
a display (27), which is configured to display multiple pixels (66, 66a, 77, 77a, 77b) of an image (55, 55a) of an organ (26) having a cavity (33) and tissue (35) surrounding the cavity; and
a processor (39), which is configured to:
receive an image signal of at least the cavity and the tissue and one or more position signals in the organ indicative of one or more positions of one or more catheters (22, 17) having a known geometry, respectively; and
**characterized in that** the system is further configured to, based on the one or more position signals, the known geometry, and the image signal: (i) identify in the image a given pixel (77a) at a given position, and (ii) display the given pixel as: (a) a first pixel indicative of the cavity responsively to identifying that the given position corresponds to the one or more positions, or (b) a second pixel indicative of the tissue.

2. The system according to claim 1, wherein the processor is configured to assign a first color to the first pixel and a second color to the second pixel, different from the first color.

3. The system according to claim 2, wherein, in response to identifying that a distal end of one of the catheters fails to reach an additional position, the processor is configured to identify an additional pixel, which corresponds to the additional position, and to assign the second color to the additional pixel.

4. A method, comprising:
displaying multiple pixels (66, 66a, 77, 77a, 77b) of an image (55, 55a) of an organ (26) having a cavity (33) and tissue surrounding the cavity;
receiving an image signal of at least the cavity and the tissue and one or more position signals in the organ indicative of one or more positions of one or more catheters (22, 17) having a known geometry, respectively; and
**characterized by**:
based on the one or more position signals, the known geometry, and the image signal: (i) identifying in the image a given pixel (77a) at a given position, and (ii) displaying the given pixel as: (a) a first pixel indicative of the cavity responsively to identifying that the given position corresponds to the one or more positions, or (b) a second pixel indicative of the tissue.

5. The system according to any of claims 1-3 or the method according to claim 4, wherein the image signal is an ultrasound (US) signal.

6. The system according to claim 1 or claim 5, or the method according to claim 4 or claim 5, wherein the multiple pixels, the given pixel, and the first and second pixels comprise volumetric pixels (voxels).

7. The method according to claim 4 or claim 5, wherein displaying the image comprises assigning a first color to the first pixel and a second color to the second pixel, different from the first color, optionally wherein, in response to identifying that the distal end fails to reach an additional position, the processor is configured to identify an additional pixel, which corresponds to the additional position, and to assign the second color to the additional pixel.

8. The system according to claim 1 or claim 5, or the method according to claim 4 or claim 5, wherein the one or more catheters comprise one or both of: (i) a mapping catheter configured to sense electrical signals in the tissue, and (ii) a ablation catheter configured to apply ablation signals to the tissue.

9. The system according to claim 5, or the method according to claim 5, wherein at least a catheter among the catheters comprising a distal end having the known geometry and comprising ultrasound transducers (UT) for applying US waves to the organ and producing the US signal at a respective position of the distal end.

10. The system according to claim 9 or the method according to claim 9, where the catheter comprises a four-dimensional (4D) ultrasound catheter, wherein the UT are arranged in a two-dimensional (2D) array at the distal end, and where the position sensor is coupled to the distal end at a known location relative to the 2D array.

11. The system according to claim 10, wherein the processor is configured to calibrate between a first coordinate system of the 2D array and a second coordinate system of the position sensor, and to identify the given pixel in the image based on the calibrated first and second coordinate systems.

12. The system according to claim 9, wherein, when the distal end is moved within the cavity between a first position and a second position, different from the first position, the processor is configured to receive: (i) first and second position signals indicative of the first and second positions, respectively, and (ii) first and second US signals indicative of the cavity at the first and second positions, respectively, and, based on the first and second position signals and US signals, to present on the display at least first and second US images of the first and second positions, respectively.

13. The method according to claim 10, and comprising calibrating between a first coordinate system of the 2D array and a second coordinate system of the position sensor, and identifying the given pixel in the image based on the calibrated first and second coordinate systems.

14. The method according to claim 9, wherein, when the distal end is moved within the cavity between a first position and a second position, different from the first position, receiving: (i) first and second position signals indicative of the first and second positions, respectively, and (ii) first and second US signals indicative of the cavity at the first and second positions, respectively, and, based on the first and second position signals and US signals, presenting on the display at least first and second US images of the first and second positions, respectively.

15. The system according to claim 12 or the method according to claim 14, wherein the first and second US images comprise first and second three-dimensional (3D) US images, respectively.

## Patentansprüche

1. System, umfassend:
eine Anzeige (27), die konfiguriert ist, um mehrere Pixel (66, 66a, 77, 77a, 77b) eines Bildes (55, 55a) eines Organs (26) anzuzeigen, das einen Hohlraum (33) und den Hohlraum umgebendes Gewebe (35) aufweist; und
einen Prozessor (39), der konfiguriert ist zum:
Empfangen eines Bildsignals mindestens des Hohlraums und des Gewebes und eines oder mehrerer Positionssignale in dem Organ, die eine bzw. mehrere Positionen eines oder mehrerer Katheter (22, 17) mit einer bekannten Geometrie anzeigen; und
**dadurch gekennzeichnet, dass** das System ferner konfiguriert ist zum, basierend auf dem einen oder den mehreren Positionssignalen, der bekannten Geometrie und dem Bildsignal,
(i) Identifizieren eines gegebenen Pixels (77a) an einer gegebenen Position in dem Bild, und (ii) Anzeigen des gegebenen Pixels als: (a) ein erstes Pixel, das den Hohlraum anzeigt, als Reaktion auf das Identifizieren, dass die gegebene Position der einen oder den mehreren Positionen entspricht, oder (b) ein zweites Pixel, das das Gewebe anzeigt.

2. System nach Anspruch 1, wobei der Prozessor konfiguriert ist, um dem ersten Pixel eine erste Farbe und dem zweiten Pixel eine zweite Farbe, die von der ersten Farbe verschieden ist, zuzuweisen.

3. System nach Anspruch 2, wobei der Prozessor als Reaktion auf das Identifizieren, dass ein distales Ende eines der Katheter eine zusätzliche Position nicht erreicht, konfiguriert ist, um ein zusätzliches Pixel zu identifizieren, das der zusätzlichen Position entspricht, und dem zusätzlichen Pixel die zweite Farbe zuzuweisen.

4. Verfahren, umfassend:
Anzeigen mehrerer Pixel (66, 66a, 77, 77a, 77b) eines Bildes (55, 55a) eines Organs (26) mit einem Hohlraum (33) und den Hohlraum umgebendem Gewebe;
Empfangen eines Bildsignals mindestens des Hohlraums und des Gewebes und eines oder mehrerer Positionssignale in dem Organ, die eine bzw. mehrere Positionen eines oder mehrerer Katheter (22, 17) anzeigen, die eine bekannte Geometrie aufweisen; und
**gekennzeichnet durch**: basierend auf dem einen oder den mehreren Positionssignalen, der bekannten Geometrie und dem Bildsignal: (i) Identifizieren eines gegebenen Pixels (77a) an einer gegebenen Position in dem Bild, und (ii) Anzeigen des gegebenen Pixels als: (a) ein erstes Pixel, das den Hohlraum anzeigt, als Reaktion auf das Identifizieren, dass die gegebene Position der einen oder den mehreren Positionen entspricht, oder (b) ein zweites Pixel, das das Gewebe anzeigt.

5. System nach einem der Ansprüche 1 bis 3 oder Verfahren nach Anspruch 4, wobei das Bildsignal ein Ultraschallsignal (US-Signal) ist.

6. System nach Anspruch 1 oder 5 oder Verfahren nach Anspruch 4 oder 5, wobei die mehreren Pixel, das gegebene Pixel und die ersten und zweiten Pixel volumetrische Pixel (Voxel) umfassen.

7. Verfahren nach Anspruch 4 oder 5, wobei das Anzeigen des Bildes das Zuweisen einer ersten Farbe zu dem ersten Pixel und einer zweiten Farbe zu dem zweiten Pixel, die sich von der ersten Farbe unterscheidet, umfasst, optional wobei, als Reaktion auf das Identifizieren, dass das distale Ende eine zusätzliche Position nicht erreicht, der Prozessor konfiguriert ist, um ein zusätzliches Pixel zu identifizieren, das der zusätzlichen Position entspricht, und dem zusätzlichen Pixel die zweite Farbe zuzuweisen.

8. System nach Anspruch 1 oder 5 oder Verfahren nach Anspruch 4 oder 5, wobei der eine oder die mehreren Katheter eines oder beides von Folgendem umfassen: (i) einen Mapping-Katheter, der konfiguriert ist, um elektrische Signale in dem Gewebe zu erfassen, und (ii) einen Ablationskatheter, der konfiguriert ist, um Ablationssignale an das Gewebe anzulegen.

9. System nach Anspruch 5 oder Verfahren nach Anspruch 5, wobei mindestens ein Katheter unter den Kathetern ein distales Ende mit der bekannten Geometrie umfasst und Ultraschallwandler (UT) zum Anlegen von US-Wellen an das Organ und zum Erzeugen des US-Signals an einer jeweiligen Position des distalen Endes umfasst.

10. System nach Anspruch 9 oder Verfahren nach Anspruch 9, wobei der Katheter einen vierdimensionalen (4D) Ultraschallkatheter umfasst, wobei die UT in einem zweidimensionalen (2D) Array a dem distalen Ende angeordnet sind, und wobei der Positionssensor an einer bekannten Position relativ zu dem 2D-Array gekoppelt an das distale Ende ist.

11. System nach Anspruch 10, wobei der Prozessor konfiguriert ist, um zwischen einem ersten Koordinatensystem des 2D-Arrays und einem zweiten Koordinatensystem des Positionssensors zu kalibrieren und das gegebene Pixel in dem Bild basierend auf dem kalibrierten ersten und dem kalibrierten zweiten Koordinatensystem zu identifizieren.

12. System nach Anspruch 9, wobei, wenn das distale Ende innerhalb des Hohlraums zwischen einer ersten Position und einer zweiten Position, die von der ersten Position verschieden ist, bewegt wird, der Prozessor konfiguriert ist zum Empfangen von: (i) ersten und zweiten Positionssignalen, die die ersten bzw. zweiten Positionen anzeigen, und (ii) ersten und zweiten US-Signalen, die den Hohlraum an den ersten bzw. zweiten Positionen anzeigen, und, basierend auf den ersten und zweiten Positionssignalen und US-Signalen, Darstellen mindestens erster und zweiter US-Bilder der ersten bzw. zweiten Positionen auf der Anzeige.

13. Verfahren nach Anspruch 10, und umfassend das Kalibrieren zwischen einem ersten Koordinatensystem des 2D-Arrays und einem zweiten Koordinatensystem des Positionssensors und das Identifizieren des gegebenen Pixels in dem Bild basierend auf den kalibrierten ersten und zweiten Koordinatensystemen.

14. Verfahren nach Anspruch 9, wobei, wenn das distale Ende innerhalb des Hohlraums zwischen einer ersten Position und einer zweiten Position, die von der ersten Position verschieden ist, bewegt wird, Empfangen von: (i) ersten und zweiten Positionssignalen, die die ersten bzw. zweiten Positionen anzeigen, und (ii) ersten und zweiten US-Signalen, die den Hohlraum an den ersten bzw. zweiten Positionen anzeigen, und, basierend auf den ersten und zweiten Positionssignalen und US-Signalen, Darstellen mindestens erster und zweiter US-Bilder der ersten bzw. zweiten Positionen auf der Anzeige.

15. System nach Anspruch 12 oder Verfahren nach Anspruch 14, wobei die ersten und zweiten US-Bilder erste bzw. zweite dreidimensionale (3D) US-Bilder umfassen.

## Revendications

1. Système, comprenant :
un affichage (27), qui est configuré pour afficher de multiples pixels (66, 66a, 77, 77a, 77b) d'une image (55, 55a) d'un organe (26) ayant une cavité (33) et du tissu (35) entourant la cavité ; et
un processeur (39) qui est configuré pour :
recevoir un signal d'image au moins de la cavité et du tissu et un ou plusieurs signaux de position dans l'organe indiquant une ou plusieurs positions d'un ou plusieurs cathéters (22, 17) ayant une géométrie connue, respectivement ; et
**caractérisé en ce que** le système est configuré en outre pour, en fonction du ou des signaux de position, de la géométrie connue et du signal d'image
(i) identifier dans l'image un pixel donné (77a) à une position donnée, et (ii) afficher le pixel donné en tant que : (a) un premier pixel indiquant la cavité en réponse à l'identification que la position donnée correspond à la ou aux positions, ou (b) un second pixel indiquant le tissu.

2. Système selon la revendication 1, dans lequel le processeur est configuré pour attribuer une première couleur au premier pixel et une seconde couleur au second pixel, différente de la première couleur.

3. Système selon la revendication 2, dans lequel, en réponse à l'identification qu'une extrémité distale de l'un des cathéters ne parvient pas à atteindre une position supplémentaire, le processeur est configuré pour identifier un pixel supplémentaire, qui correspond à la position supplémentaire, et pour attribuer la seconde couleur au pixel supplémentaire.

4. Procédé, comprenant :
l'affichage de multiples pixels (66, 66a, 77, 77a, 77b) d'une image (55, 55a) d'un organe (26) ayant une cavité (33) et du tissu entourant la cavité ;
la réception d'un signal d'image au moins de la cavité et du tissu et d'un ou plusieurs signaux de position dans l'organe indiquant une ou plusieurs positions d'un ou plusieurs cathéters (22, 17) ayant une géométrie connue, respectivement ; et
**caractérisé par** : en fonction du ou des signaux de position, de la géométrie connue et du signal d'image : (i) l'identification dans l'image d'un pixel donné (77a) à une position donnée, et (ii) l'affichage du pixel donné en tant que : (a) un premier pixel indiquant la cavité en réponse à l'identification que la position donnée correspond à la ou aux positions, ou (b) un second pixel indiquant le tissu.

5. Système selon l'une quelconque des revendications 1 à 3 ou procédé selon la revendication 4, dans lequel le signal d'image est un signal d'ultrasons (US).

6. Système selon la revendication 1 ou la revendication 5, ou procédé selon la revendication 4 ou la revendication 5, dans lequel les multiples pixels, le pixel donné et les premier et second pixels comprennent des pixels volumétriques (voxels).

7. Procédé selon la revendication 4 ou la revendication 5, dans lequel l'affichage de l'image comprend l'attribution d'une première couleur au premier pixel et d'une seconde couleur au second pixel, différente de la première couleur, facultativement dans lequel, en réponse à l'identification que l'extrémité distale ne parvient pas à atteindre une position supplémentaire, le processeur est configuré pour identifier un pixel supplémentaire, qui correspond à la position supplémentaire, et pour attribuer la seconde couleur au pixel supplémentaire.

8. Système selon la revendication 1 ou la revendication 5, ou procédé selon la revendication 4 ou la revendication 5, dans lequel le ou les cathéters comprennent l'un et/ou l'autre parmi : (i) un cathéter de cartographie configuré pour capter des signaux électriques dans le tissu, et (ii) un cathéter d'ablation configuré pour appliquer des signaux d'ablation au tissu.

9. Système selon la revendication 5, ou procédé selon la revendication 5, dans lequel au moins un cathéter parmi les cathéters comprend une extrémité distale ayant la géométrie connue et comprenant des transducteurs à ultrasons (UT) permettant d'appliquer des ondes US à l'organe et de produire le signal US au niveau d'une position respective de l'extrémité distale.

10. Système selon la revendication 9 ou procédé selon la revendication 9, où le cathéter comprend un cathéter à ultrasons en quatre dimensions (4D), dans lequel les UT sont agencés dans un réseau à deux dimensions (2D) au niveau de l'extrémité distale, et où le capteur de position est accouplé à l'extrémité distale au niveau d'une localisation connue par rapport au réseau 2D.

11. Système selon la revendication 10, dans lequel le processeur est configuré pour étalonner entre un premier système de coordonnées du réseau 2D et un second système de coordonnées du capteur de position, et pour identifier le pixel donné dans l'image en fonction des premier et second systèmes de coordonnées étalonnés.

12. Système selon la revendication 9, dans lequel, lorsque l'extrémité distale est déplacée au sein de la cavité entre une première position et une seconde position, différente de la première position, le processeur est configuré pour recevoir : (i) des premier et second signaux de position indiquant les première et seconde positions, respectivement, et (ii) des premier et second signaux US indiquant la cavité au niveau des première et seconde positions, respectivement, et, en fonction des premier et second signaux de position et des signaux US, pour présenter sur l'affichage au moins des première et seconde images US des première et seconde positions, respectivement.

13. Procédé selon la revendication 10, et comprenant l'étalonnage entre un premier système de coordonnées du réseau 2D et un second système de coordonnées du capteur de position, et l'identification du pixel donné dans l'image en fonction des premier et second systèmes de coordonnées étalonnés.

14. Procédé selon la revendication 9, dans lequel, lorsque l'extrémité distale est déplacée au sein de la cavité entre une première position et une seconde position, différente de la première position, la réception : (i) de premier et second signaux de position indiquant les première et seconde positions, respectivement, et (ii) de premier et second signaux US indiquant la cavité au niveau des première et seconde positions, respectivement, et, en fonction des premier et second signaux de position et des signaux US, la présentation sur l'affichage au moins des première et seconde images US des première et seconde positions, respectivement.

15. Système selon la revendication 12 ou procédé selon la revendication 14, dans lequel les première et seconde images US comprennent des première et seconde images US en trois dimensions (3D), respectivement.
